# EUROPÄISCHE PATENTSCHRIFT

(11) **EP 2 096 098 B1**
(45) Veröffentlichungstag und Bekanntmachung des Hinweises auf die Patenterteilung: **15.07.2015**
(21) Anmeldenummer: 09150380.5
(22) Anmeldetag: 10.02.2003
(51) Int. Cl.: C07C 29/149, B01J 23/80, B01J 35/10, B01J 35/02

(54) **Katalysator zur Herstellung von Alkoholen**
Catalyst for the production of alcohols
Catalyseur pour la préparation d'alcools

(30) Priorität: 22.02.2002 DE 10207443
(43) Veröffentlichungstag der Anmeldung: 02.09.2009
(62) Teilanmeldung aus: 03002604.1
(73) Patentinhaber: LANXESS Deutschland GmbH, 50569 Köln (DE)
(72) Erfinder: Prinz, Thomas, 51371 Leverkusen (DE); Kintrup, Jürgen, 51373 Leverkusen (DE); Schulze Tilling, Andreas, League City, TX 77573 (US); Jentsch, Jörg-Dietrich, 51381 Leverkusen (DE); John, Gerald, 40477 Düsseldorf (DE); Gross, Hans-Jürgen, 42929 Wermelskirchen (DE); Giffels, Guido, 53177 Bonn (DE)

(56) Entgegenhaltungen:
- EP-A- 0 424 069
- EP-A- 0 901 815
- WO-A-02/081416
- DE-A- 19 942 895
- US-A- 4 393 251
- US-A- 5 019 547
- US-A- 5 155 086
- US-A- 5 334 779
- US-A- 5 345 005
- US-A- 5 453 412
- US-A- 6 124 234

## Beschreibung

Die vorliegende Erfindung betrifft Katalysatoren zur Herstellung von Alkoholen durch Umsetzung von Carbonsäuren und/oder Carbonsäureestern mit Wasserstoff in Gegenwart dieser Katalysatoren.

Die Hydrierung von Carbonsäuren und Carbonsäureestern in Gegenwart von Katalysatoren auf Basis von Kupferchromit (Adkins-Katalysator) ist seit langem bekannt. Die Verwendung von chromhaltigen Katalysatoren ist jedoch aus Gründen der damit verbundenen Gefahren für die Umwelt nicht wünschenswert. Es sind daher Anstrengungen unternommen worden, diese chromhaltigen Katalysatoren durch umweltfreundlichere chromfreie Katalysatoren zu ersetzen.

So ist aus WO 82/03854 A1 ein Verfahren zur Hydrierung von Carbonsäureestern bekannt, wobei in Gegenwart eines Katalysators gearbeitet wird, der eine reduzierte Mischung aus Kupferoxid und Zinkoxid enthält.

EP-A 0 721 928 beschreibt ein Verfahren zur Herstellung von aliphatischen α,ω-Diolen durch Hydrierung von Carbonsäureestern mit einem Katalysator, der eine reduzierte Mischung von verpressten Pulvern von Kupfer-, Zink- und Aluminiumoxiden, denen Eisen-, Nickel- oder Manganoxid zugesetzt sein kann, enthält. US-A 5 155 086 beschreibt pulverförmige Hydrierkatalysatoren auf Kupfer/Zink/- Aluminium-Basis, die eine Hauptmenge an Oxiden des Kupfers und Zinks und geringere Mengen an Aluminiumoxid enthalten, wobei das Porenvolumen der Poren, die einen Durchmesser zwischen 120 und 1000 Å aufweisen, mindestens 40 % des gesamten Porenvolumens beträgt. Insbesondere eignen sich die Katalysatoren zur Hydrierung von Aldehyden, Ketonen, Carbonsäuren und Carbonsäureestern. Katalysatoren basierend auf Kupfer/Zink/Aluminium sind aus der Methanolsynthese bekannt (US-A 4 305 842, EP 125 689 A2). Aluminiumoxid und Zinkoxid haben bei diesen Katalysatoren die Funktion eines Trägermaterials für Kupfer. Derartige Katalysatoren werden beispielsweise durch Kofällung der Komponenten hergestellt und durch Kalzinierung und Reduktion zu aktiven Katalysatoren umgewandelt (Knözinger, Ertl, Weitkamp, Handbook of Catalysis, VCH Wiley, Weinheim 1997, 1836).

Es sind auch Hydrierkatalysatoren auf Basis von Kupfer/Zink/Aluminium bekannt, bei denen kolloidales TiO₂ oder Al(OH)₃ zum Produkt der Kofällung von Kupfer und Zink zugesetzt wurden (EP 125 689 A2, Petrini et al., Preparation of catalysts III, Studies in surface science and catalysis, 16, Elsevier, Amsterdam, 1983, 735-755.)

JP J09-173845 beschreibt die Herstellung von Cu/Zn-Katalysatoren, die durch die Tränkung von γ-Aluminiumoxiden hergestellt werden, wobei der Aluminiumgehalt bei ca. 53 % liegt und die Verwendung solcher Katalysatoren in der Synthese von Dimethylether.

WO 99/4974 beschreibt Katalysatoren, die durch die Auffällung von Cu und Zn auf TiO₂ hergestellt werden. Zur Herstellung von Tabletten aus dem pulverförmigen Katalysator wird als Tablettierhilfsmittel metallisches Kupferpulver zugesetzt, um eine ausreichende Härte zu erzielen. In DE 19942895 ist ebenfalls die Wirkung von metallischem Kupfer oder Zement als Tablettierhilfsmittel zur Herstellung von Cu/Zn/Al-Katalysatoren zur Hydrierung von Carbonsäureestern beschrieben. Auch hier führt dieser Zusatz zu einer Erhöhung der Seitenbruchhärte.

WO 97/34694 beschreibt Cu/Al/Zn- Katalysatoren mit einem Al-Gehalt größer 20 %, die in Form von Extrudaten eine bimodale Porengrößenverteilung haben. Diese Katalysatoren sind gut geeignet für die Hydrierung von Fettsäureestern.

Die bekannten Katalysatoren werden beim Einsatz in Festbettreaktoren als Formkörper eingesetzt, die bei den dort auftretenden mechanischen Belastungen nur begrenzte mechanische Stabilität aufweisen. Zudem ist die Hydrieraktivität dieser Katalysatoren, besonders bei der Hydrierung von Estern mehrwertiger Säuren mit mehrwertigen Alkoholen, beispielsweise Gemischen oligomerer Ester aus Adipinsäure und Hexandiol, für die Erreichung hoher Raum-Zeit-Ausbeuten unzureichend. EP-A 0 424 069 und US-A 5,345,005 beschreibt u.a. Katalysatoren, enthaltend Kupfer, Zink und Aluminium mit einer spezifischen Oberfläche von 50 m²/g bzw. mindestens 70 m²/g und einer sehr inhomogenen Korngrößenverteilung. Diese weisen nachteiligerweise nur eine geringe Produktselektivität und kurze Standzeiten auf.

Aufgabe der Erfindung war es daher, einen Katalysator für die Herstellung von Alkoholen durch Umsetzung von Carbonsäuren und/oder Carbonsäureestern mit Wasserstoff zur Verfügung zu stellen, wobei sich der Katalysator unter den Reaktionsbedingungen durch eine hohe mechanische Stabilität und eine hohe Aktivität auszeichnet, so dass höhere Raum-Zeit-Ausbeuten erzielt werden.

Überraschend wurde gefunden, dass man Katalysatoren mit hoher mechanischer Stabilität und hoher Aktivität erhält, wenn man Verbindungen von Kupfer und Zink gemeinsam auf ein Trägermaterial aus Aluminiumoxidpulver auffällt und die anschließende Calzinierung in der Weise durchführt, dass sich eine bimodale Porengrößenverteilung einstellt.

Gegenstand der Erfindung ist einen Katalysator für die Herstellung von Alkoholen durch Umsetzung von Carbonsäuren und/oder Carbonsäureestern mit Wasserstoff zur Verfügung zu stellen, wobei der Katalysator in nichtreduziertem Zustand 20 bis 80 Gew.-% CuO, 10 bis 80 Gew.-% ZnO und 1 bis 50 Gew.-% Al₂O₃ enthält und eine Porengrößenverteilung derart hat, dass 5 bis 15 % des Gesamtporenvolumens im Porendurchmesserbereich kleiner 15 nm [150 Å] und 80 bis 95 % im Porendurchmesserbereich größer 25 nm [250 Å] liegen mit einer spezifischen Oberfläche, bestimmt nach BET, analog zu DIN 66131, von 5 bis 60 m²/g. Die Porengrößenverteilung wurde dabei durch Quecksilberintrusion (Quecksilberporosimetrie) analog zu DIN 66133 unter Annahme eines zylindrischen Porenmodells bestimmt wird.

Die erfindungsgemäß einzusetzenden Katalysatoren werden bevorzugt durch Auffällung von Verbindungen des Kupfers und Zinks auf Aluminiumoxidpulver hergestellt.

Die erfindungsgemäß einzusetzenden Katalysatoren zeichnen sich insbesondere durch eine hohe Hydrieraktivität und eine besondere mechanische und chemische Stabilität, insbesondere beim Einsatz in Festbettreaktoren mit Flüssigfahrweise aus.

Vorzugsweise werden Katalysatoren verwendet, die in nichtreduziertem Zustand 40 bis 70 Gew.-% CuO, 20 bis 50 Gew.-% ZnO und 4 bis 10 Gew.-% Al₂O₃ enthalten.

Besonders bevorzugt sind Katalysatoren, die im nichtreduzierten Zustand 60 bis 70 Gew.-% CuO, 20 bis 27 Gew.-% ZnO und 4 bis 6 Gew.-% Al₂O₃ enthalten.

Die Katalysatoren können zusätzlich Verbindungen der Seltenerdmetalle, Alkalimetalle, Erdalkalimetalle, Zr, Ti, Co, Mo, V, W, Fe, Co, Ni, Mn, Re in einer Menge von 0,1 bis 3 Gew.-% als Promotoren enthalten.

Das Gesamtporenvolumen liegt bevorzugt im Bereich von 100 bis 350 mm³/g, besonders bevorzugt bei 150 mm³/g bis 250 mm³/g.

Die spezifische Oberfläche (bestimmt nach BET, analog zu DIN 66131 durch Stickstoffadsorption bei -196°C) der Katalysatoren in nicht reduziertem Zustand liegt bei 5 bis 150 m²/g, bevorzugt bei 5 bis 60 m²/g und besonders bevorzugt bei 5 bis 30 m²/g.

Bevorzugt wird dabei Aluminiumoxidpulver mit einer Partikelgröße (mittlerer Partikeldurchmesser) von 1 bis 100 µm, besonders bevorzugt 3 bis 80 µm, insbesondere bevorzugt 10 bis 50 µm eingesetzt.

Die spezifische Oberfläche des Aluminiumoxidpulvers (bestimmt nach BET) beträgt vorzugsweise 100 bis 400 m²/g, besonders bevorzugt 100 bis 300 m²/g, das Porenvolumen bevorzugt 0,1 bis 1,5 ml/g, besonders bevorzugt 0,4 bis 0,8 ml/g.

Der Natriumgehalt des Aluminiumoxidpulvers beträgt vorteilhafterweise 0 bis 2 Gew.-%, bevorzugt 0,01 bis 0,1 Gew.-%.

Analog zum Aluminiumoxidpulver können auch pulverförmige Mischoxide des Aluminiums, zum Beispiel die des Siliziums mit den gleichen physikalischen Eigenschaften eingesetzt werden.

Nach dem Verfahren werden Alkohole durch Hydrierung von Carbonsäureestern und/oder Carbonsäuren erhalten. Die Einsatzstoffe können in technischer Qualität eingesetzt werden.

Besonders vorteilhaft lassen sich nach dem Verfahren zweiwertige Alkohole durch Hydrierung von zweiwertigen Carbonsäuren und/oder deren Ester mit den entsprechenden Alkoholen erhalten, wobei ein Teil des Einsatzmaterials aus höhermolekularen Estern bestehen kann.

Besonders bevorzugt werden als Edukt Gemische oligomerer Ester aus Adipinsäure und Hexandiol eingesetzt. Als Produkt wird in diesem Fall 1,6-Hexandiol erhalten. Vorzugsweise wird bei einer Temperatur von 100 bis 350°C, besonders bevorzugt bei 150 bis 300°C und insbesondere bevorzugt bei 200 bis 280°C gearbeitet.

Der Druck, beim dem Verfahren durchgeführt wird, beträgt bevorzugt 50 bis 400 bar, besonders bevorzugt 100 bis 300 bar.

Die Umsetzung kann beispielsweise in einem Suspensionsreaktor durchgeführt werden. In diesem Fall wird der Katalysator pulverförmig eingesetzt. Bevorzugt liegt der Katalysator als Pulver mit einer Partikelgröße (mittlerer Partikeldurchmesser) von 20 bis 100 µm vor.

Die Umsetzung kann aber beispielsweise auch in einem Festbettreaktor erfolgen, wobei der Katalysator zweckmäßigerweise als Formkörper verwendet wird.

Es kann in einem Reaktor oder auch in mehreren hintereinandergeschalteten Reaktoren gearbeitet werden.

Das Verfahren kann sowohl ohne als auch mit Zusatz von Lösungsmitteln, z.B. Alkoholen, durchgeführt werden.

Bei der Umsetzung von Carbonsäuren mit Wasserstoff ist es vorteilhaft, die Hydrierung in einem Alkohol als Lösungsmittel durchzuführen.

Geeignete Alkohole sind beispielsweise Methanol, Ethanol, Isopropanol, n-Propanol, Butandiol oder Hexandiol. Bevorzugt setzt man den Alkohol als Lösungsmittel ein, der durch die Hydrierung der Carbonsäure entsteht.

Weiterhin sind Katalysatoren für die Herstellung von Alkoholen durch Umsetzung von Carbonsäuren und/oder Carbonsäureestern mit Wasserstoff Gegenstand der Erfindung, die dadurch gekennzeichnet sind, dass sie in nichtreduziertem Zustand 20 bis 80 Gew.-% CuO, 10 bis 80 Gew.-% ZnO und 1 bis 50 Gew.-% Al₂O₃ enthalten und durch Auffällung von Verbindungen des Kupfers und Zinks auf Aluminiumoxidpulver hergestellt werden und eine Porengrößenverteilung derart haben, dass 5 bis 15 % des Gesamtporenvolumens im Porendurchmesserbereich kleiner (150 Å ) 15 nm und 80 bis 95 % im Porendurchmesserbereich größer ( 250 Å ) 25 nm liegen, mit einer spezifischen Oberfläche, bestimmt nach BET, analog zu DIN 66131, von 5 bis 60 m2/g. Die Porengrößenverteilung wurde dabei durch Quecksilberintrusion (Quecksilberporosimetrie) analog zu DIN 66133 unter Annahme eines zylindrischen Porenmodells bestimmt·

Bevorzugte Ausführungsformen des Katalysators entsprechen dem, was bereits bei der Beschreibung des Verfahrens ausgeführt wurde.

Die Herstellung der erfindungsgemäßen Katalysatoren kann beispielsweise folgendermaßen erfolgen: Aluminiumoxidpulver wird in Wasser suspendiert und auf eine Temperatur von 20 bis 90°C, bevorzugt 50 bis 80°C temperiert. Aus einem Vorlagebehälter wird eine wässrige Lösung mit der Konzentration von 0,1 bis 3 mol/l, bevorzugt 0,5 bis 1,5 mol/l an Kupfersalz, bevorzugt Kupfernitrat, und eine entsprechende Menge Zinksalz, bevorzugt Zinknitrat, zu dem suspendierten Aluminiumoxidpulver gepumpt. Das molare Verhältnis von Kupfer zu Zink, berechnet als Metall, beträgt dabei 8:1 bis 1:4, bevorzugt 3,5:1 bis 1:1,25 und besonders bevorzugt 3,5:1 bis 2,2:1. Gleichzeitig wird eine wässrige Lösung mit der Konzentration von 0,1 bis 5 mol/l, bevorzugt 0,5 bis 2 mol/l einer Base, bevorzugt Ammoniumcarbonat, Natriumcarbonat, Natronlauge oder Mischungen derselben, zugepumpt. Man stellt die Zugabegeschwindigkeit der beiden Lösungen so ein, dass der pH-Wert bei der Temperatur, bei der die Fällung vorgenommen wird, innerhalb des Bereichs von 5,9 bis 9, bevorzugt 5,9 bis 8,1, gehalten wird. Die Fällung wird bei einer möglichst konstanten Temperatur im Bereich von 20 bis 90°C, bevorzugt 50 bis 80°C durchgeführt. Im Anschluss an die Fällung lässt man die entstandene Suspension bei einer Temperatur von 20 bis 90°C, bevorzugt 70 bis 90°C über einen Zeitraum von 0,5 bis 3 Stunden nachrühren. Danach wird filtriert und mit Wasser, bevorzugt bei 15 bis 70°C, besonders bevorzugt bei 15 bis 25°C gewaschen. Der Filterkuchen wird beispielsweise bei Temperaturen von 70 bis 150°C, ggf. im Vakuum getrocknet. Die Trocknung kann auch gleichzeitig mit einer Sprühagglomeration, z.B. in einem Sprühtrockner, zu Partikeln mit einem weitgehend einheitlichen Durchmesser, vorzugsweise im Bereich von 10 bis 80 µm durchgeführt werden. Das getrocknete Material wird anschließend über einen Zeitraum von 2 bis 6 Stunden bei einer Temperatur im Bereich von 300 bis 900°C kalziniert. Soll der Katalysator in Pulverform eingesetzt werden, so ist eine Kalzinierung im Bereich 400°C bis 800°C bevorzugt und im Bereich 450°C bis 700°C besonders bevorzugt. Soll das Material für den Einsatz im Festbettreaktor z.B. durch Tablettierung oder Extrudierung agglomeriert werden, so ist eine Kalzinierung bei 300°C bis 600°C bevorzugt und bei 300°C bis 500°C besonders bevorzugt.

Der kalzinierte Katalysator kann beispielsweise im Hydrierreaktor, in dem die erfindungsgemäße Umsetzung erfolgt, mit Wasserstoff reduziert werden. Es ist auch möglich, den kalzinierten Katalysator in einem separaten Reduktionsofen zu reduzieren. Soll der Katalysator in einem Suspensionsreaktor eingesetzt werden, wird der Katalysator zweckmäßig in Form des Pulvers eingesetzt.

Für den Einsatz in einem Festbettreaktor ist es vorteilhaft, den Katalysator einer Formgebung, z.B. durch Tablettierung oder Extrudierung zu unterziehen. Hierzu können beispielsweise Hilfsmittel, z.B. Graphit, Magnesiumstearat, Zinkstearat in einer Menge von 0,5 bis 5 Gew.-% zugesetzt werden. Bei einer Formgebung durch Tablettierung wird durch die Apparateeinstellung vorzugsweise eine Seitenbruchhärte von 30 bis 250 N, bevorzugt mehr als 33 N, besonders bevorzugt von 100 bis 200 N eingestellt. Das kalzinierte Pulver kann auch vor der Formgebung reduziert werden. Nach der Formgebung kann eine zusätzliche Kalzinierung zur weiteren Erhöhung der mechanischen Festigkeit und Verbesserung der chemischen Eigenschaften zum Beispiel bei 300°C bis 900°C oder 400°C bis 900°C, bevorzugt bei 450°C bis 800°C und besonders bevorzugt bei 450°C bis 700°C erfolgen.

In einer besonderen Ausführungsform der Erfindung kann dem Pulver vor der Kompaktierung auch ein Porenbildner zugesetzt werden, der durch eine nachfolgende Calzinierung zu einer zusätzlichen Bildung von Poren führt. Als Porenbildner kann beispielsweise das hier beschriebene unkalzinierte getrocknete Fällprodukt aus Kupfer- und Zinksalzen auf Aluminiumoxid eingesetzt werden.

Im folgenden wird die Erfindung anhand von Beispielen weiter erläutert.

### Beispiele

### Beispiel 1 (Vergleich) Katalysatorherstellung

72 g Aluminiumoxidpulver (spezifische Oberfläche 146,5 m²/g) wird in einem Fällkessel in 4 1 Wasser suspendiert und auf 70°C temperiert. Aus einem Vorlagebehälter werden 15 kg einer wässrigen Lösung, enthaltend 2628 g Cu(NO₃)₂ x 2,5 H₂O und 1200 g Zn(NO₃)₂ x 6 H₂O innerhalb von 3 Stunden in den Fällkessel gepumpt. Gleichzeitig wird eine wässrige Sodalösung mit der Konzentration von 1 mol/l zugepumpt. Man stellt die Zugabegeschwindigkeit der Sodalösung so ein, dass der pH-Wert innerhalb des Bereichs von 6,8 bis 7 gehalten wird. Die Fällung wird bei einer Temperatur von 70°C durchgeführt. Im Anschluss an die Fällung lässt man die Suspension bei 70°C über einen Zeitraum von 2 Stunden nachrühren. Danach wird filtriert und mit Wasser gewaschen. Der Filterkuchen wird 12 Stunden bei 120°C im Vakuum getrocknet. Das getrocknete Material wird anschließend über einen Zeitraum von 4 Stunden bei 400°C kalziniert. Das Kalzinierprodukt wird gemahlen, mit 5 Gew.-% Graphit vermischt und auf einer Tablettenpresse zu Zylindern mit einer Höhe von 5 mm und einem Durchmesser von 5 mm tablettiert. Die Seitenbruchhärte wird auf 117 N eingestellt. Die spezifische Oberfläche (BET) beträgt 41,2 m²/g und wurde analog zu DIN 66131 bestimmt. Die Seitenbruchhärte im reduzierten Zustand beträgt 78 N. Das Gesamtporenvolumen beträgt 188,8 mm³/g. Die Porengrößenverteilung ist derart, dass 17,9 % des Gesamtporenvolumens im Porendurchmesserbereich kleiner 150 Å und 41,4 % des Gesamtporenvolumens im Porendurchmesserbereich größer 250 Å sind. Die genaue Porengrößenverteilung ist in Tabelle 1 dargestellt.

### Beispiel 2 Katalysatorherstellung

Der Katalysator aus Beispiel 1 wird als fertige, oxidische Tablette 4 h lang bei 480°C nachkalziniert. Die Seitenbruchhärte beträgt 300 N. Die spezifische Oberfläche (BET) beträgt 26,4 m²/g. Die Seitenbruchhärte im reduzierten Zustand beträgt 50 N. Das Gesamtporenvolumen beträgt 211,2 mm³/g. Die Porengrößenverteilung ist derart, dass 11,1 % des Gesamtporenvolumens im Porendurchmesserbereich kleiner 150 Å und 84,9 % des Gesamtporenvolumens im Porendurchmesserbereich größer 250 Å sind. Die genaue Porengrößenverteilung ist in Tabelle 1 dargestellt.

### Beispiel 3 Katalysatorherstellung

Die Herstellung erfolgt analog Beispiel 1, jedoch wird das Pulver vor der Kompaktierung 4 h bei 480°C kalziniert. Anschließend werden 5 % Graphit zugesetzt und zu Tabletten mit einem Durchmesser von 5 mm und einer Höhe von 3 mm tablettiert. Die Seitenbruchhärte beträgt 121 N. Die spezifische Oberfläche (BET) beträgt 24,0 m²/g. Die Seitenbruchhärte im reduzierten Zustand beträgt 47 N. Das Gesamtporenvolumen beträgt 191,9 mm³/g. Die Porengrößenverteilung ist derart, dass 12,3 % des Gesamtporenvolumens im Porendurchmesserbereich kleiner 150 Å und 81,5 % des Gesamtporenvolumens im Porendurchmesserbereich größer 250 Å sind. Die genaue Porengrößenverteilung ist in Tabelle 1 dargestellt.

### Beispiel 4 (Vergleich) Katalysatorherstellung

72 g Aluminiumoxidpulver (spezifische Oberfläche 146,5 m²/g) wird in einem Fällkessel in 4 1 Wasser suspendiert und auf 60°C temperiert. Aus einem Vorlagebehälter werden 15 kg einer wässrigen Lösung, enthaltend 2628 g Cu(NO₃)₂ x 2,5 H₂O und 1200 g Zn(NO₃)₂ x 6 H₂O innerhalb von 3 Stunden in den Fällkessel gepumpt. Gleichzeitig wird eine wässrige Sodalösung mit der Konzentration von 1 mol/l zugepumpt. Man stellt die Zugabegeschwindigkeit der Sodalösung so ein, dass der pH-Wert innerhalb des Bereichs von 5,9 bis 6,1 gehalten wird. Die Fällung wird bei einer Temperatur von 60°C durchgeführt. Im Anschluss an die Fällung lässt man die Suspension bei 60°C über einen Zeitraum von 2 Stunden nachrühren. Danach wird filtriert und mit Wasser gewaschen. Der Filterkuchen wird 12 Stunden bei 120°C im Vakuum getrocknet. Das getrocknete Material wird anschließend über einen Zeitraum von 4 Stunden bei 400°C kalziniert. Das Kalzinierprodukt wird gemahlen, mit 5 Gew.-% Graphit vermischt und auf einer Tablettenpresse zu Zylindern mit einer Höhe von 5 mm und einem Durchmesser von 5 mm tablettiert. Die Seitenbruchhärte wird auf 110N eingestellt. Die spezifische Oberfläche (BET) beträgt 56,4 m²/g. Die Seitenbruchhärte im reduzierten Zustand beträgt 36 N. Das Gesamtporenvolumen beträgt 240,0 mm³/g. Die Porengrößenverteilung ist derart, dass 15,9 % des Gesamtporenvolumens im Porendurchmesserbereich kleiner 150 Å und 21,6 % des Gesamtporenvolumens im Porendurchmesserbereich größer 250 Å sind. Die genaue Porengrößenverteilung ist in Tabelle 1 dargestellt.

### Beispiel 5 (Vergleich) Katalysatorherstellung

Der Katalysator aus Beispiel 4 wird als fertige, oxidische Tablette 4 h lang bei 700°C nachkalziniert. Die Seitenbruchhärte beträgt 350 N. Die spezifische Oberfläche (BET) beträgt 7,0 m²/g. Das Gesamtporenvolumen beträgt 112,0 mm³/g. Die Porengrößenverteilung ist derart, dass 17,2 % des Gesamtporenvolumens im Porendurchmesserbereich kleiner 150 Å und 79,2 % des Gesamtporenvolumens größer 250 Å sind. Die genaue Porengrößenverteilung ist in Tabelle 1 dargestellt.

### Beispiel 6 (Vergleich) Katalysatorherstellung

61,5 g Aluminiumoxidpulver (spezifische Oberfläche 146,5 m²/g) wird in einem Fällkessel in 4 1 Wasser suspendiert und auf 70°C temperiert. Aus einem Vorlagebehälter werden 12,8 kg einer wässrigen Lösung, enthaltend 2234 g Cu(NO₃)₂ x 2,5 H₂O und 896 g Zn(NO₃)₂ x 6 H₂O innerhalb von 6 Stunden in den Fällkessel gepumpt. Gleichzeitig wird eine wässrige Sodalösung mit der Konzentration von 1 mol/l zugepumpt. Man stellt die Zugabegeschwindigkeit der Sodalösung so ein, dass der pH-Wert innerhalb des Bereichs von 7,9 bis 8,1 gehalten wird. Die Fällung wird bei einer Temperatur von 70°C durchgeführt. Im Anschluss an die Fällung lässt man die Suspension bei 70°C über einen Zeitraum von 2 Stunden nachrühren. Danach wird filtriert und mit Wasser gewaschen. Der Filterkuchen wird 12 Stunden bei 120°C im Vakuum getrocknet. Das getrocknete Material wird anschließend über einen Zeitraum von 4 Stunden bei 350°C kalziniert. Das Kalzinierprodukt wird gemahlen, mit 5 Gew.-% Graphit vermischt und auf einer Tablettenpresse zu Zylindern mit einer Höhe von 5 mm und einem Durchmesser von 5 mm tablettiert. Die Seitenbruchhärte wird auf 176 N eingestellt. Die spezifische Oberfläche (BET) beträgt 57,3 m²/g. Die Seitenbruchhärte im reduzierten Zustand beträgt 34 N. Das Gesamtporenvolumen beträgt 165,8 mm³/g. Die Porengrößenverteilung ist derart, dass 53,8 % des Gesamtporenvolumens im Porendurchmesserbereich kleiner 150 Å und 26,8 % des Gesamtporenvolumens im Porendurchmesserbereich größer 250 Å sind. Die genaue Porengrößenverteilung ist in Tabelle 1 dargestellt.

### Beispiel 7 Katalysatorherstellung

Der Katalysator aus Beispiel 6 wird als fertige, oxidische Tablette 4 h lang bei 600°C nachkalziniert. Die Seitenbruchhärte beträgt 158 N. Die spezifische Oberfläche (BET) beträgt 15,4 m²/g. Das Gesamtporenvolumen beträgt 214,4 mm³/g. Die Porengrößenverteilung ist derart, dass 10,2 % des Gesamtporenvolumens im Porendurchmesserbereich kleiner 150 Å und 88,9 % des Gesamtporenvolumens im Porendurchmesserbereich größer 250 Å sind. Die genaue Porengrößenverteilung ist in Tabelle 1 dargestellt.

**Tabelle 1:**

| Porendurchmesserintervall | Relatives Hg-Porenvolumen [mm³/g] Katalysator aus Beispiel 1 | Relatives Hg-Porenvolumen [%] Katalysator aus Beispiel 1 | Relatives Hg-Porenvolumen [mm³/g] Katalysator aus Beispiel 2 | Relatives Hg-Porenvolumen [%] Katalysator aus Beispiel 2 | Relatives Hg-Porenvolumen [mm³/g] Katalysator aus Beispiel 3 | Relatives Hg-Porenvolumen [%] Katalysator aus Beispiel 3 | Relatives Hg-Porenvolume n [mm³/g] Katalysator aus Beispiel 4 | Relatives Hg-Porenvolumen [%] Katalysator aus Beispiel 4 |
|---|---|---|---|---|---|---|---|---|
| 2000000 Å -1000000 Å | 0 | 0 | 0 | 0 | 0,00 | 0,00 | 0 | 0 |
| 1000000 Å - 500000 A | 0,27 | 0,14 | 0,78 | 0,37 | 0,00 | 0,00 | 0,82 | 0,34 |
| 500000 Å - 200000 Å | 0,41 | 0,21 | 0,49 | 0,23 | 0,38 | 0,20 | 0,47 | 0,19 |
| 200000 Å - 100000 Å | 0,81 | 0,43 | 0,29 | 0,14 | 0,19 | 0,10 | 0,35 | 0,15 |
| 100000 Å - 50000 Å | 1,22 | 0,64 | 0,39 | 0,18 | 0,38 | 0,20 | 0,58 | 0,24 |
| 50000 Å - 20000 Å | 0,81 | 0,43 | 0,19 | 0,09 | 0,38 | 0,20 | 0,35 | 0,15 |
| 20000 Å - 10000 Å | 0,00 | 0,00 | 0,10 | 0,05 | 0,00 | 0,00 | 0,12 | 0,05 |
| 10000 Å - 5000 Å | 0,00 | 0,00 | 0,39 | 0,18 | 0,00 | 0,00 | 0,35 | 0,15 |
| 5000 Å - 2000 Å | 0,00 | 0,00 | 4,18 | 1,98 | 0,76 | 0,39 | 0,82 | 0,34 |
| 2000 Å - 1000 Å | 4,32 | 2,29 | 21,87 | 10,36 | 14,19 | 7,40 | 1,64 | 0,68 |
| 1000 Å - 600 Å | 19,32 | 10,24 | 39,56 | 18,74 | 29,71 | 15,48 | 3,04 | 1,27 |
| 600 Å - 400 Å | 18,38 | 9,74 | 52,98 | 25,09 | 54,68 | 28,50 | 6,20 | 2,58 |
| 400 Å - 300 Å | 15,95 | 8,45 | 46,37 | 21,96 | 42,57 | 22,19 | 11,46 | 4,77 |
| 300 Å - 250 Å | 16,62 | 8,80 | 11,57 | 5,48 | 13,06 | 6,80 | 25,72 | 10,72 |
| 250 Å - 200 Å | 37,16 | 19,69 | 4,96 | 2,35 | 7,19 | 3,75 | 74,46 | 31,03 |
| 200 Å - 150 Å | 39,73 | 21,05 | 3,60 | 1,70 | 4,73 | 2,47 | 75,51 | 31,47 |
| 150 Å - 100 Å | 22,03 | 11,67 | 11,18 | 5,29 | 12,30 | 6,41 | 28,05 | 11,69 |
| 100 Å - 50 Å | 10,27 | 5,44 | 11,08 | 5,25 | 9,27 | 4,83 | 8,65 | 3,60 |
| 50 Å - 37 Å | 1,49 | 0,79 | 1,17 | 0,55 | 2,08 | 1,08 | 1,40 | 0,58 |
| **Summe** | **188,79** | **100** | **211,15** | **100** | **191,87** | **100** | **239,99** | **100** |

| Porendurchmesserintervall | Relatives Hg-Porenvolumen [mm³/g] Katalysator aus Beispiel 5 | Relatives Hg-Porenvolumen [%] Katalysator aus Beispiel 5 | Relatives Hg-Porenvolumen [mm³/gl Katalysator aus Beispiel 6 | Relatives Hg-Porenvolumen [%] Katalysator aus Beispiel 6 | Relatives Hg-Porenvolumen [mm³/g] Katalysator aus Beispiel 7 | Relatives HgPorenvolumen [%] Katalysator aus Beispiel 7 | | |
|---|---|---|---|---|---|---|---|---|
| 2000000 Å- 1000000 Å | 0,00 | 0,00 | 0 | 0 | 0 | 0 | | |
| 1000000 Å - 500000 Å | 0,18 | 0,16 | 0,46 | 0,28 | 0,40 | 0,19 | | |
| 500000 Å - 200000 Å | 0,00 | 0,00 | 0,58 | 0,35 | 0,40 | 0,19 | | |
| 200000 Å - 100000 Å | 0,00 | 0,00 | 0,35 | 0,21 | 0,13 | 0,06 | | |
| 100000 Å - 50000 Å | 0,45 | 0,40 | 0,00 | 0,00 | 0,13 | 0,06 | | |
| 50000 Å - 20000 Å | 1,18 | 1,05 | 0,23 | 0,14 | 1,20 | 0,56 | | |
| 20000 Å - 10000 Å | 1,09 | 0,97 | 0,12 | 0,07 | 7,71 | 3,60 | | |
| 10000 Å - 5000 Å | 7,89 | 7,04 | 0,35 | 0,21 | 17,01 | 7,94 | | |
| 5000 Å - 2000 Å | 25,02 | 22,35 | 1,27 | 0,76 | 24,72 | 11,53 | | |
| 2000 Å - 1000 Å | 39,26 | 35,06 | 6,67 | 4,02 | 57,02 | 26,60 | | |
| 1000 Å - 600 Å | 10,52 | 9,39 | 11,27 | 6,80 | 46,52 | 21,70 | | |
| 600 Å - 400 Å | 1,54 | 1,38 | 10,24 | 6,18 | 24,99 | 11,66 | | |
| 400 Å - 300 Å | 0,82 | 0,73 | 7,71 | 4,65 | 8,77 | 4,09 | | |
| 300 Å - 250 Å | 0,73 | 0,65 | 5,18 | 3,12 | 1,59 | 0,74 | | |
| 250 Å - 200 Å | 1,18 | 1,05 | 8,17 | 4,93 | 0,93 | 0,43 | | |
| 200 Å - 150 Å | 2,90 | 2,59 | 24,05 | 14,50 | 1,06 | 0,50 | | |
| 150 Å - 100 Å | 10,97 | 9,80 | 57,52 | 34,70 | 6,51 | 3,04 | | |
| 100 Å - 50 Å | 7,80 | 6,96 | 31,64 | 19,08 | 15,28 | 7,13 | | |
| 50 Å - 37 Å | 0,45 | 0,40 | 0,00 | 0,00 | 0,00 | 0,00 | | |
| **Summe** | **111,98** | **100** | **165,81** | **100** | **214,37** | **100** | | |

### Beispiele 8 bis 14 Katalysatoranwendung

Ein senkrecht stehendes, wärmeisoliertes Hochdruckrohr aus nichtrostendem, säurefestem Material von 45 mm Durchmesser und 1 m Länge, das vorher mit Stickstoff sauerstofffrei gespült wurde, wird mit 1,4 1 der Katalysatoren nach Beispiel 1 bis 7 gefüllt. Zur Aktivierung des Katalysators wird zunächst 6 Stunden lang bei 200°C ein Stickstoffstrom (5 Nm³/h) durch das Katalysatorbett geleitet. Nun erfolgt die Reduktion des Katalysators, indem bei einem Stickstoffdruck von 200 bar und einer Temperatur zwischen 180°C und 200°C langsam Wasserstoff zugemischt wird, wobei der Anfangsgehalt 10 bis 15 Vol.-% nicht übersteigen sollte. Über den Zeitraum von 24 h wird der Stickstoffanteil mehr und mehr vermindert, bis letztlich reiner Wasserstoff durch den Reaktor strömt. Die Reaktion ist beendet, wenn kein Reaktionswasser mehr gebildet wird.

Nach der Aktivierung des Katalysators wird der Wasserstoffdruck auf 300 bar erhöht und ein Volumenstrom von 5 Nm³/h eingestellt. Nun wird Hexandiol-1,6-adipat durch den Reaktor gefördert, das durch Veresterung von Adipinsäure mit 1,6-Hexandiol im Verhältnis 1:1,1 erhalten wurde (vgl. EP-A 0 721 928). Die Zulaufmenge und die entsprechende Temperatur ist der nachfolgenden Tabelle 2 zu entnehmen. Jedes in der Tabelle 2 angegebene Wertepaar aus Zulaufmenge und Temperatur wird dabei mindestens für 48 h eingestellt. Das aus dem Reaktionsrohr austretende Reaktionsgemisch wird in einem zweiten Wärmeaustauscher (Wasserkühler) bei 300 bar Wasserstoffdruck auf kleiner 60°C gekühlt und in einem Gasabscheider von überschüssigem Wasserstoff, der wieder in das Hydriersystem zurückgeführt wird, getrennt. Nach weiterer Abkühlung auf eine Temperatur kleiner 30°C und Entspannung auf Normaldruck wird das Reaktionsprodukt gaschromatographisch untersucht. Die Rohausbeute an 1,6-Hexandiol ist in der Tabelle 2 ebenfalls angegeben.

**Tabelle 2:**

| **Beispiel** | **Katalysator** | **Zulaufmenge Ester** | **Temperatur** | **Rohausbeute 1,6-Hexandiol** |
|---|---|---|---|---|
| Vergleichsbeispiel 8 | aus Beispiel 1 | 200 ml/h | 240 °C | 96,7 % |
| | | 400 ml/h | 240 °C | 84,6% |
| | | 600 ml/h | 240 °C | 77,7 % |
| | | 600 ml/h | 260 °C | 80,6% |
| | | 400 ml/h | 260 °C | 81,7 % |
| Der Versuch wurde nach einer Gesamtlaufzeit von 2080 h beendet. Der Katalysator war zu diesem Zeitpunkt praktisch noch unverändert wirksam. | | | | |
| Beispiel 9 | aus Beispiel 2 | 200 ml/h | 240 °C | 94,9% |
| | | 400 ml/h | 240 °C | 94,0% |
| | | 600 ml/h | 240 °C | 89,2% |
| | | 600 ml/h | 260 °C | 87,4% |
| | | 400 ml/h | 260 °C | 92,4% |
| Der Versuch wurde nach einer Gesamtlaufzeit von 900 h beendet. Der Katalysator war zu diesem Zeitpunkt praktisch noch unverändert wirksam. | | | | |
| Beispiel 10 | aus Beispiel 3 | 200 ml/h | 240 °C | 97,3% |
| | | 400 ml/h | 240 °C | 97,5% |
| | | 600 ml/h | 240°C | 96,6% |
| | | 600 ml/h | 260 °C | 93,0% |
| | | 400 ml/h | 260 °C | 91,9% |
| | | 900 ml/h | 260 °C | 93,4% |
| | | 1000 ml/h | 260 °C | 91,7% |
| Der Versuch wurde nach einer Gesamtlaufzeit von 3412 h beendet. Der Katalysator war zu diesem Zeitpunkt praktisch noch unverändert wirksam. | | | | |
| Vergleichsbeispiel 11 | aus Beispiel 4 | 200 ml/h | 240 °C | 96,2 % |
| | | 400 ml/h | 240 °C | 80,5% |
| | | 600 ml/h | 240 °C | 73,1 % |
| | | 600 ml/h | 260 °C | 74,7% |
| | | 400 ml/h | 260 °C | 85,0% |
| Der Versuch wurde nach einer Gesamtlaufzeit von 526 h beendet. Der Katalysator war zu diesem Zeitpunkt praktisch noch unverändert wirksam. | | | | |
| Vergleichsbeispiel 12 | aus Beispiel 5 | 200 ml/h | 240 °C | 90,6 % |
| | | 400 ml/h | 240 °C | 93,0% |
| | | 600 ml/h | 240 °C | 83,6% |
| | | 600 ml/h | 260 °C | 86,3% |
| | | 400 ml/h | 260 °C | 91,3% |
| Der Versuch wurde nach einer Gesamtlaufzeit von 862 h beendet. Der Katalysator war zu diesem Zeitpunkt praktisch noch unverändert wirksam. | | | | |
| Vergleichsbeispiel 13 | aus Beispiel 6 | 200 ml/h | 240°C | 97,5 % |
| | | 400 ml/h | 240°C | 89,2% |
| | | 600 ml/h | 240°C | 85,3% |
| | | 600 ml/h | 260 °C | 85,4% |
| | | 400 ml/h | 260 °C | 88,3% |
| Der Versuch wurde nach einer Gesamtlaufzeit von 520 h beendet. Der Katalysator war zu diesem Zeitpunkt praktisch noch unverändert wirksam. | | | | |
| Beispiel 14 | aus Beispiel 7 | 200 ml/h | 240°C | 97,9% |
| | | 400 ml/h | 240°C | 97,3% |
| | | 600 ml/h | 240°C | 96,5% |
| | | 600 ml/h | 260 °C | 96,1 % |
| | | 800 ml/h | 240 °C | 93,7% |
| | | 800 ml/h | 250 °C | 95,8% |
| | | 1000 ml/h | 260 °C | 94,7% |
| | | 1100 ml/h | 260 °C | 93,1% |
| Der Versuch wurde nach einer Gesamtlaufzeit von 642 h beendet. Der Katalysator war zu diesem Zeitpunkt praktisch noch unverändert wirksam. | | | | |

## Patentansprüche

1. Katalysator für die Herstellung von Alkoholen durch Umsetzung von Carbonsäuren und/oder Carbonsäureestern mit Wasserstoff, **dadurch gekennzeichnet, dass** der Katalysator in nichtreduziertem Zustand
• 20 bis 80 Gew.-% CuO,
• 10 bis 80 Gew.-% ZnO und
• 1 bis 50 Gew.-% Al₂O₃ enthält und
• eine Porengrößenverteilung aufweist, bei der 5 bis 15 % des Gesamtporenvolumens im Porendurchmesserbereich kleiner 15 nm [150 Å] und 80 bis 95 % im Porendurchmesserbereich größer 25 nm [250 Å] liegen und
• eine spezifische Oberfläche, bestimmt nach BET analog zu DIN 66131, von 5 bis 60 m²/g besitzt.

2. Katalysatoren nach Anspruch 1, **dadurch gekennzeichnet, dass** sie durch Auffällung von Verbindungen des Zinks und des Kupfers auf Aluminiumoxidpulver hergestellt werden.

3. Katalysatoren nach einem der Ansprüche 1 bis 2, **dadurch gekennzeichnet, dass** sie ein Gesamtporenvolumen von 100 bis 350 mm³/g haben.

4. Katalysatoren nach einem der Ansprüche 1 bis 3, **dadurch gekennzeichnet, dass** sie in Form der Tabletten im reduzierten Zustand ein Seitenbruchhärte von mehr als 33 N haben.

5. Katalysatoren nach einem der Ansprüche 1 bis 4, **dadurch gekennzeichnet, dass** sie hergestellt werden, indem man Kupfer und Zink aus ihren Salzen auf Aluminiumoxidpulver auffällt, das Produkt abfiltriert und nachwäscht, anschließend bei einer Temperatur von 70 bis 150°C trocknet, bei einer Temperatur von 300°C bis 600°C kalziniert und anschließend tablettiert und die erhaltenen Tabletten ggf. bei einer Temperatur von 400°C bis 900°C nachkalziniert.

6. Katalysatoren nach Anspruch 5, **dadurch gekennzeichnet, dass** man dem Pulver vor der Tablettierung einen Porenbildner zusetzt und nach der Tablettierung bei einer Temperatur von 300°C bis 900°C nachkalziniert.

## Claims

1. Catalyst for the preparation of alcohols by reacting carboxylic acids and/or carboxylic esters with hydrogen, **characterized in that** the catalyst in the non-reduced state comprises
• 20 to 80% by weight of CuO,
• 10 to 80% by weight of ZnO and
• 1 to 50% by weight of Al₂O₃, and
• has a pore size distribution in which 5 to 15% of the total pore volume is in the pore diameter range of less than 15 nm [150 Å] and 80 to 95% is in the pore diameter range of greater than 25 nm [250 Å] and
• has a specific surface area, determined according to BET analogously to DIN 66131, of 5 to 60 m²/g.

2. Catalysts according to Claim 1, **characterized in that** they are obtained by precipitating compounds of zinc and of copper onto aluminium oxide powder.

3. Catalysts according to one of Claims 1 to 2, **characterized in that** they have a total pore volume of 100 to 350 mm³/g.

4. Catalysts according to one of Claims 1 to 3, **characterized in that** when in the form of tablets in the reduced state, they have a lateral fracture hardness of more than 33 N.

5. Catalysts according to one of Claims 1 to 4, **characterized in that** they are prepared by precipitating copper and zinc from their salts onto aluminium oxide powder, filtering and washing the product, then drying it at a temperature of 70 to 150°C, calcining it at a temperature of 300°C to 600°C and then tableting it and optionally further calcining the tablets obtained at a temperature of 400°C to 900°C.

6. Catalysts according to Claim 5, **characterized in that** a pore former is added to the powder before tableting and, after tableting, further calcining is effected at a temperature of 300°C to 900°C.

## Revendications

1. Catalyseur pour la fabrication d'alcools par mise en réaction d'acides carboxyliques et/ou d'esters d'acides carboxyliques avec de l'hydrogène, **caractérisé en ce que** le catalyseur contient à l'état non réduit :
- 20 à 80 % en poids de CuO,
- 10 à 80 % en poids de ZnO et
- 1 à 50 % en poids d'Al₂O₃, et
- présente une distribution des tailles de pores selon laquelle 5 à 15 % du volume poreux total se situe dans la plage de diamètres de pores de moins de 15 nm [150 Å] et 80 à 95 % dans la plage de diamètres de pores de plus de 25 mm [250 Å], et
- possède une surface spécifique, déterminée selon BET de manière analogue à DIN 66131, de 5 à 60 m²/g.

2. Catalyseurs selon la revendication 1, **caractérisés en ce qu'**ils sont fabriqués par précipitation de composés de zinc et de cuivre sur une poudre d'oxyde d'aluminium.

3. Catalyseurs selon l'une quelconque des revendications 1 à 2, **caractérisés en ce qu'**ils présentent un volume poreux total de 100 à 350 mm³/g.

4. Catalyseurs selon l'une quelconque des revendications 1 à 3, **caractérisés en ce qu'**ils présentent sous la forme de pastilles à l'état réduit une résistance à la rupture latérale de plus de 33 N.

5. Catalyseurs selon l'une quelconque des revendications 1 à 4, **caractérisés en ce qu'**ils sont fabriqués par précipitation de cuivre et de zinc à partir de leurs sels sur une poudre d'oxyde d'aluminium, filtration et lavage du produit, puis séchage à une température de 70 à 150 °C, calcination à une température de 300 °C à 600 °C, puis pastillage, et éventuellement post-calcination des pastilles obtenues à une température de 400 °C à 900 °C.

6. Catalyseurs selon la revendication 5, **caractérisés en ce qu'**un agent de formation de pores est ajouté à la poudre avant le pastillage et une post-calcination est réalisée après le pastillage à une température de 300 °C à 900 °C.
